# FASCICULE DE BREVET EUROPEEN

(11) **EP 1 094 759 B1**
(45) Date de publication et mention de la délivrance du brevet: **28.07.2004**
(21) Numéro de dépôt: 99915846.2
(22) Date de dépôt: 27.04.1999
(51) Int. Cl.: A61B 17/70

(54) **DISPOSITIF D'OSTEOSYNTHESE RACHIDIENNE POUR FIXATION ANTERIEURE AVEC PLAQUE**
WIRBELSÄULE-OSTEOSYNTHESEVORRICHTUNG ZUR VORDEREN FIXIERUNG MITTELS EINER PLATTE
BACKBONE OSTEOSYNTHESIS DEVICE FOR ANTERIOR FIXING WITH PLATE

(30) Priorité: 06.07.1998 FR 9808602
(43) Date de publication de la demande: 02.05.2001
(73) Titulaire: Dimso Distribution Medicale du Sud Ouest, 33610 Cestas (FR)
(72) Inventeur: MANGIONE, Paolo, F-33600 Pessac (FR); LE COUEDIC, Régis, F-33610 Cestas (FR); PASQUET, Denis, F-33000 Bordeaux (FR); CONCHY, Frédéric, F-33650 Saint-Médard-d'Eyrans (FR)
(74) Mandataire: Le Forestier, Eric
(86) Numéro de dépôt international: PCT/FR1999/000994
(87) Numéro de publication internationale: WO 2000/001314

(56) Documents cités:
- EP-A- 0 556 548
- WO-A-94/06360
- WO-A-94/20048
- FR-A- 2 651 992
- FR-A- 2 766 353

## Description

L'invention concerne les systèmes d'ostéosynthèse rachidienne, notamment pour fixation antérieure.

On connaît d'après le document WO 94/06360 un système d'ostéosynthèse du rachis pour fixation antérieure. Ce système comporte une plaque longitudinale et un bras d'un seul tenant avec la plaque, s'étendant à une extrémité de celle-ci en étant incliné par rapport à sa direction longitudinale. Ce bras reçoit deux vis à os à ancrer dans une vertèbre. L'autre extrémité de la plaque présente deux orifices oblongs parallèles entre eux destinés à recevoir deux vis à os respectives à ancrer dans une autre vertèbre. Les deux vertèbres de fixation sont séparées par la vertèbre à arthrodéser. Les orifices oblongs permettent le réglage de la position longitudinale des deux vis par rapport au bras. Le nombre de pièces à assembler étant réduit, ce système est rapide à monter lors d'une intervention. Toutefois, la pièce principale de ce système est volumineuse, ce qui la rend difficile à introduire dans le corps par voie endoscopique. De plus, cette pièce principale n'est pas adaptée à être conformée à la configuration des vertèbres du patient en vue d'optimiser sa position ou la qualité de sa fixation aux vertèbres.

Un but de l'invention est de fournir un système à la fois plus rapide à monter, et facile à introduire par voie endoscopique.

En vue de la réalisation de ce but, on prévoit selon l'invention un système d'ostéosynthèse conforme à la revendication 1.

Ainsi, le deuxième bras étant séparable de la plaque, le système se compose d'éléments de petite taille et aptes à être introduits dans le corps par voie endoscopique comme par voie normale. Mis à part les vis, les éléments essentiels du système sont au nombre de deux, de sorte que le montage du système (assemblage et positionnement relatif des pièces) dans le corps est simple à effectuer. Le temps opératoire demeure donc court. La présence de la plaque d'un seul tenant avec le premier bras permet d'envisager la réduction par voie antérieure d'une cyphose très importante en agissant comme bras de levier sur la vertèbre associée à ce bras. De plus, on peut placer la plaque de la manière la plus postérieure possible sur le corps vertébral, ce qui dégage l'accès à la vertèbre abîmée ou au greffon. Enfin, en vue de l'adapter à la configuration des vertèbres, on peut facilement conformer le deuxième bras puisqu'il est indépendant du reste du système.

Avantageusement, l'invention pourra présenter une ou plusieurs des caractéristiques énoncées aux revendications 2 à 11.

D'autres caractéristiques et avantages de l'invention apparaîtront encore dans la description suivante d'un mode préféré de réalisation donné à titre d'exemple non limitatif. Aux dessins annexés :

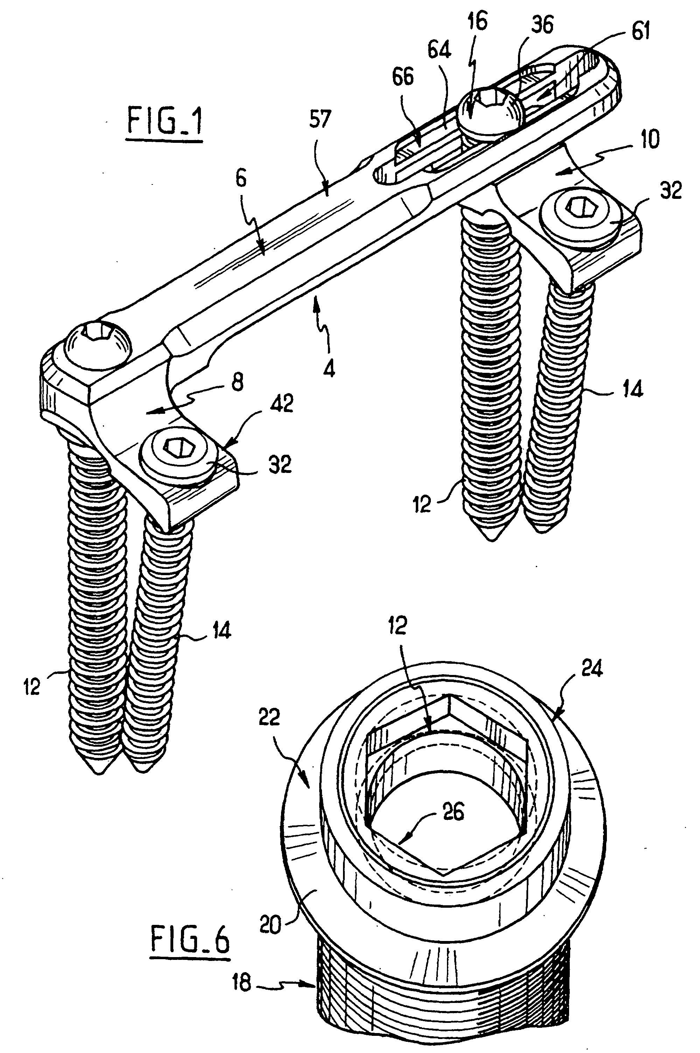

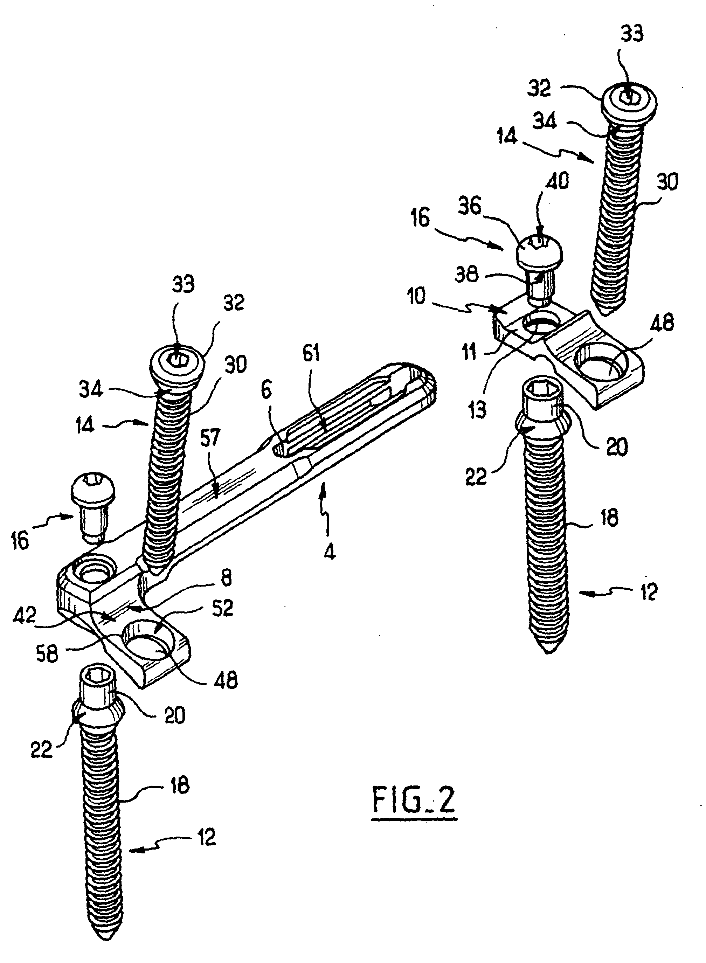

- les figures 1 et 2 sont deux vues en perspective respectivement à l'état monté et à l'état éclaté d'un mode préféré de réalisation du système d'ostéosynthèse selon l'invention ;
- les figures 3 et 4 sont deux vues en perspective respectivement de dessus et de dessous du deuxième bras du système de la figure 1 ;
- la figure 5 est une vue en perspective de l'élément constituant la plaque et le premier bras du système de la figure 1 ;
- la figure 6 est une vue en perspective de la tête de l'une des vis d'ancrage primaire ; et
- la figure 7 montre le système de la figure 1 monté sur des vertèbres.

Le système d'ostéosynthèse rachidienne pour fixation antérieure selon l'invention comporte dans le présent mode de réalisation un élément 4 d'un seul tenant formant plaque 6 et premier bras 8. Le système comporte un deuxième bras 10 et pour chaque bras 8, 10, deux vis d'ancrage vertébral primaire 12 et secondaire 14 ainsi qu'une vis de serrage 16. Les deux vis primaires 12 sont identiques entre elles, ainsi que les deux vis secondaires 14 et les deux vis de serrage 16.

On notera que les vis secondaires peuvent en pratique être identiques ou différentes, selon le choix du chirurgien.

Chaque vis primaire 12 a un corps cylindrique fileté 18. Elle est autotaraudeuse et bicorticale. Le corps est muni d'un filetage à os. La vis présente une tête 20 et, entre le corps et la tête, une collerette ayant une face supérieure 22 de forme tronconique allant en se rétrécissant en direction de la tête. En référence notamment à la figure 6, la tête 20 de la vis présente une face externe cylindrique lisse 24 et une face interne 26 de forme hexagonale à six pans dans laquelle est creusé un filet. L'empreinte à six pans permet d'actionner la vis à rotation au moyen d'un outil.

Dans une variante de réalisation, la vis est dépourvue de l'empreinte six pans mais présente seulement un filet ménagé dans une face interne cylindrique. De plus, la collerette a cette fois une forme hexagonale, ou bien présente deux méplats parallèles à l'axe de la vis, parallèles entre eux et diamétralement opposés l'un à l'autre en vue de coopérer avec une clé de serrage, ou encore tout autre moyen pour mettre la vis primaire en rotation.

Chaque vis secondaire 14 est autotaraudeuse et monocorticale (ou en variante bicorticale). Elle présente un corps cylindrique fileté 30 avec un filetage à os. Elle comprend une tête 32 présentant dans sa face supérieure une empreinte hexagonale six pans 33 ou tout autre forme apte à coopérer avec un outil de serrage tel qu'une clé ou un tournevis. La tête présente une face inférieure 34 contiguë au corps et de forme sphérique.

Chaque vis de serrage 16 a un corps cylindrique fileté apte à constituer une liaison vis-écrou avec la tête de la vis primaire associée 12. La vis de serrage 16 comprend une tête 36 ayant une face inférieure plane 38 perpendiculaire à l'axe de la vis. La tête 36 présente sur sa face supérieure une empreinte 40 du type précité, adaptée à coopérer avec un outil de serrage.

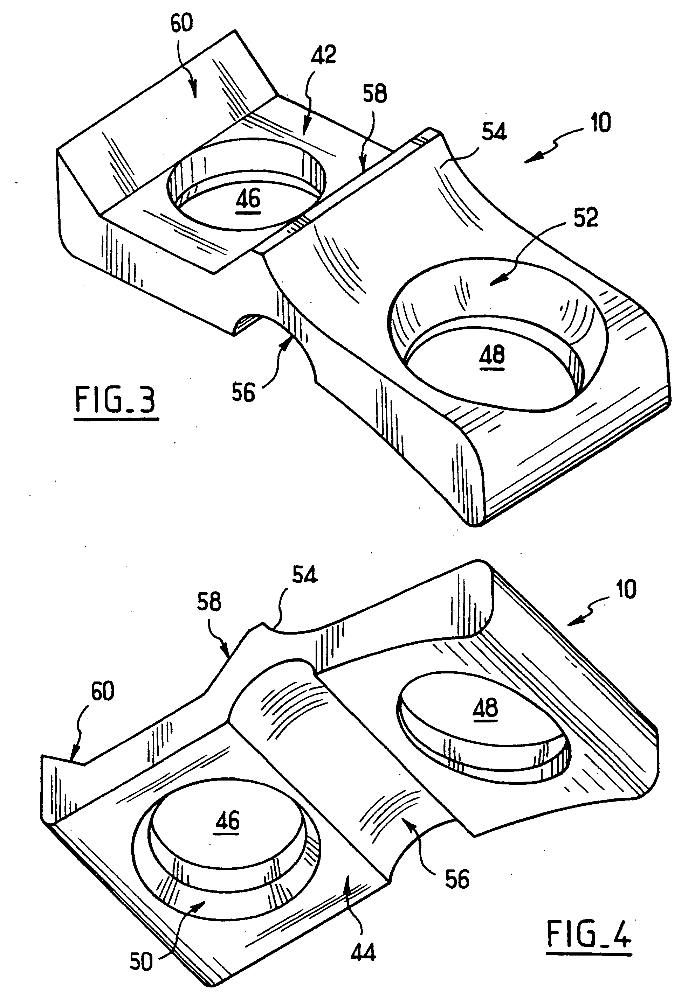

En référence aux figures 3 et 4, le deuxième bras ou connecteur 10 a une forme générale plate rectangulaire en plan définissant deux faces planes supérieure 42 et inférieure 44 généralement parallèles entre elles. Le bras présente des premier et deuxième orifices circulaires 46, 48. La face supérieure 42 sert, au voisinage du premier orifice 46, d'appui plan à la face inférieure 38 de la vis de serrage 16 associée pour réaliser un contact surfacique avec celle-ci et la bloquer à friction pour son serrage. Le bord inférieur 50 de cet orifice a une forme tronconique apte à former un contact surfacique avec la face tronconique 22 de la collerette de la vis primaire 12. Le deuxième orifice 48 a un bord supérieur 52 de forme sphérique apte à venir en contact avec la face inférieure sphérique 34 de la vis secondaire 14 en vue de régler la position de la vis secondaire par angulation dans son logement constitué par l'orifice 48.

La face supérieure 42 du deuxième bras présente une nervure 54 s'étendant entre les deux orifices 46, 48, perpendiculairement à la direction longitudinale du bras, et séparant cette face en deux parties sensiblement égales. Environ au droit de cette nervure 65, la face inférieure 44 présente une gorge profilée 56 à section transversale circulaire, plus proche du deuxième orifice 48 que du premier orifice 46, de sorte qu'elle constitue un amincissement local du bras le rendant apte à être cintré manuellement durant une intervention chirurgicale. La rainure 54 présente en regard du premier orifice 46 un chanfrein plan 58 incliné par rapport à la face 42. Le bras présente à un bord d'extrémité adjacent au premier orifice une bordure ménageant un deuxième chanfrein plan 60 incliné par rapport à la face 42, en regard du premier orifice. Les deux chanfreins 58, 60 s'étendent en regard l'un de l'autre suivant une direction transversale commune et définissent en section une forme trapézoïdale femelle.

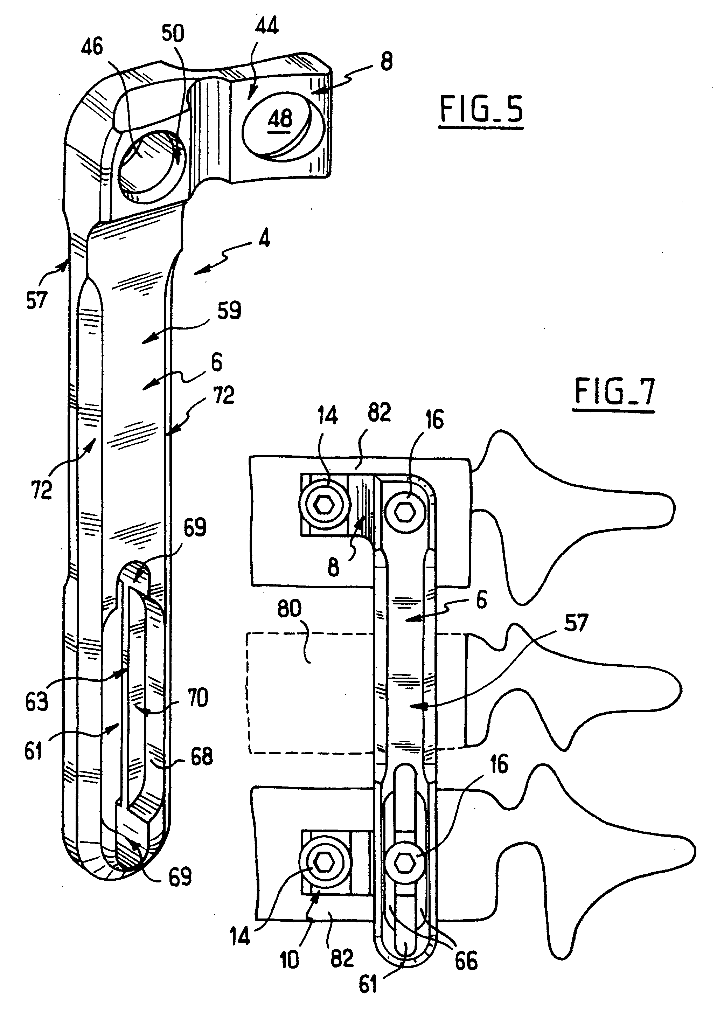

En référence à la figure 5, sur l'élément 4, le premier bras 8 est identique au deuxième bras 10 sauf que l'évidement constitué par les deux chanfreins 58, 60 et la portion de face supérieure 42 entre ceux-ci est absent. La plaque a une forme générale plate rectiligne allongée. Le premier bras 8 et la plaque 6, qui s'étendent ici perpendiculairement l'un à l'autre, donnent à l'élément 4 une forme générale en "L". Une extrémité du premier bras 8 s'étend à une première extrémité de la plaque 6.

La plaque 6 présente une face supérieure 57 et une face inférieure 59 parallèles l'une à l'autre. La plaque 6 présente un orifice oblong 60 s'étendant parallèlement à une direction longitudinale de la plaque. L'orifice a des bords plans 63 parallèles l'un à l'autre.

Sur une portion médiane de l'orifice, sur une longueur supérieure à la moitié ou aux trois-quarts de l'orifice, les bords 63 présentent des évidements respectifs 64 de part et d'autre de l'orifice, adjacents à la face supérieure 57, ménageant chacun une face plane 66 parallèle à la face supérieure et une face plane perpendiculaire à celle-ci et parallèle à la direction longitudinale de la plaque. Du côté de la face inférieure 59, la plaque est munie d'évidements 68 analogues, à faces 70, adjacents à la face inférieure 59 de la plaque. Les extrémités de l'orifice dépassant de ces évidements 64, 68 constituent des prolongements 69 de largeur inférieure à la largeur médiane de l'orifice mesurée au niveau des évidements 64, 68. La plaque 6 présente deux chanfreins plans 72 ménagés sur les bords longitudinaux de la face inférieure 59. En section transversale, ces chanfreins 72 et la face inférieure constituent une forme trapézoïdale mâle.

Lors du montage, on effectue un assemblage mâle-femelle avec les chanfreins 58, 60 du deuxième bras 10 qui reçoit par au-dessus des chanfreins 72 de la plaque, en réalisant deux à deux un contact surfacique assurant un centrage du deuxième bras par rapport à la plaque suivant la largeur de la plaque. Les quatre chanfreins sont dimensionnés de sorte qu'un interstice est ménagé, même après blocage, entre la face inférieure 59 de la plaque et la face supérieure 42 du deuxième bras qui ne viennent pas en contact. On assure ainsi une coopération selon des lignes de force obliques qui induisent une légère déformation élastique des matériaux au niveau des chanfreins en appui et donc des efforts importants. Dans ces conditions, le frottement entre les chanfreins est suffisant pour assurer un blocage stable en position longitudinale du deuxième bras par rapport à la plaque.

La tête de la vis principale 12 est introduite par en-dessous dans le premier orifice 46 du deuxième bras 10 puis par en-dessous dans l'orifice oblong 61 de la plaque. La vis de réglage 16 est engagée par au-dessus dans la tête de la vis principale 12. Sa face plane inférieure 38 vient en appui surfacique à une position quelconque contre la face plane 66 des évidements 64 pour réaliser un bon serrage. On peut ainsi choisir la position longitudinale du deuxième bras 10 le long de l'orifice oblong de la plaque 6.

La face tronconique 20 de la vis principale 12 vient en contact surfacique contre le bord tronconique 50 du deuxième bras 10 pour un blocage à fixation rigide de la vis par rapport au bras.

En référence à la figure 7, lors d'une intervention, après exposition de la vertèbre affectée 80 et des deux vertèbres adjacentes 82, on réalise une corporectomie tout en préservant, quand cela est possible, les plateaux respectifs de celles-ci. On réalise un avant-trou sur le côté latéral de la vertèbre 82 à égale distance des plateaux supérieur et inférieur, ainsi qu'à la limite du quart le plus postérieur du corps vertébral. On introduit ensuite une vis primaire 12 dans cet avant-trou jusqu'à la collerette d'arrêt. Le deuxième bras 10 est ensuite positionné sur celle-ci. Les faces tronconiques 20, 50 venant en contact mutuel, on examine la conformité du deuxième bras 10 à la vertèbre 82 associée que l'on peut modifier en retirant le deuxième bras et en le cintrant manuellement. Ensuite, on visse la vis secondaire 14 dans le deuxième orifice 48 du deuxième bras 10 jusqu'à mettre sa face sphérique 34 en contact avec celle 52 du deuxième bras. Il est préférable de positionner le deuxième bras 10 le plus parallèlement aux plateaux que possible. On fixe l'élément 4 de la même façon au moyen du premier bras 8.

Après avoir ainsi instrumenté les deux vertèbres adjacentes 82, on positionne la plaque 50 dans le deuxième bras 10. Le serrage final est effectué au moyen de la vis de serrage-réglage 16 introduite comme précité dans la vis primaire 12 du deuxième bras 10. Préalablement, on peut effectuer une contraction ou une distraction pour parfaire la distance voulue à l'aide d'une pince distractive dont les extrémités peuvent être reçues dans les prolongements 69 de l'orifice oblong 61.

## Revendications

1. Système d'ostéosynthèse du rachis pour fixation antérieure, comprenant un premier bras (8, 10) apte à recevoir des vis à os (12, 14), et une plaque longitudinale (6), la plaque (6) étant d'une seule pièce avec le premier bras (8), **caractérisé en ce qu'**il comprend un deuxième bras (8, 10) apte à recevoir des vis à os, **en ce que** la plaque longitudinale est apte à relier rigidement les bras **en ce qu'**il comprend des moyens de réglage (16, 61) d'une position longitudinale du deuxième bras (10) sur la plaque (6) et **en ce qu'**il comprend au moins une vis de fixation (16) apte à être engagée dans une des vis à os (12, 14) associée à l'un des bras (8, 10) pour fixer le bras à la vis à os.

2. Système selon la revendication 1, **caractérisé en ce que** la plaque (6) présente au moins un orifice (61) pour le réglage de la position longitudinale.

3. Système selon la revendication 2, **caractérisé en ce que** l'orifice (61) a une forme oblongue.

4. Système selon la revendication 3, **caractérisé en ce que** l'orifice (61) présente à au moins une extrémité un prolongement (69) de largeur inférieure à une largeur d'une partie médiane de l'orifice.

5. Système selon la revendication 3 ou 4, **caractérisé en ce qu'**il comporte une vis de réglage (16) apte à être fixée au deuxième bras (10) et à coulisser dans l'orifice (61).

6. Système selon l'une quelconque des revendications 1 à 5, **caractérisé en ce que** le bras (8, 10) présente un orifice (46) apte à être interposé entre la vis à os (12) et la vis de fixation (16).

7. Système selon la revendication 5, **caractérisé en ce que** la vis de fixation (16) est la vis de réglage.

8. Système selon l'une quelconque des revendications 1 à 7, **caractérisé en ce que** le deuxième bras (10) et la plaque (6) présentent des faces de centrage (58, 60, 72) aptes à venir en contact mutuel pour centrer le bras par rapport à la plaque.

9. Système selon la revendication 8, **caractérisé en ce que** le bras (10) et la plaque (6) présentent chacun deux faces de centrage aptes à former un assemblage mâle-femelle.

10. Système selon la revendication 8 ou 9, **caractérisé en ce que** les faces de centrage (58, 60, 72) sont planes.

11. Système selon l'une quelconque des revendications 1 à 10, **caractérisé en ce que** chaque bras (8, 10) est apte à être cintré manuellement.

## Patentansprüche

1. Wirbelsäule-Osteosynthesevorrichtung zur vorderen Fixierung, welche einen ersten Arm (8, 10) aufweist, in den Knochenschrauben (12, 14) eingesetzt werden können und eine längsförmige Platte (6), wobei diese Platte (6) einteilig mit dem ersten Arm (8) ausgebildet ist,
**dadurch gekennzeichnet, dass**
sie einen zweiten Arm (8, 10) aufweist, in den Knochenschrauben eingesetzt werden können, und dass die längsförmige Platte die Arme steif miteinander verbinden kann, und dass diese Vorrichtung Mittel (16, 61) für die Regulierung der Längsrichtung des zweiten Armes (10) auf der Platte (6) aufweist und dass sie mindestens eine Befestigungsschraube (16) enthält, die in eine der an einem der Arme (8, 19) angeordneten Knochenschrauben (12, 14) eingesetzt werden kann, um diesen Arm an der Knochenschraube zu befestigen.

2. Wirbelsäule-Osteosynthesevorrichtung Anspruch 1,
**dadurch gekennzeichnet, dass**
die Platte (6) mindestens eine Öffnung (61) für die Regulierung der Position in Längsrichtung aufweist.

3. Wirbelsäule-Osteosynthesevorrichtung nach Anspruch 2,
**dadurch gekennzeichnet, dass**
diese Öffnung (61) eine längliche Form hat.

4. Wirbelsäule-Osteosynthesevorrichtung nach Anspruch 3,
**dadurch gekennzeichnet, dass**
die Öffnung (61) mindestens an einem ihrer Enden eine Verlängerung (69) mit einer Breite aufweist, die geringer ist, als die Breite des mittleren Teils dieser Öffnung.

5. Wirbelsäule-Osteosynthesevorrichtung nach einem der Ansprüche 3 oder 4,
**dadurch gekennzeichnet, dass**
sie eine Stellschraube (16) aufweist, die an dem zweiten Arm (10) befestigt ist und in der Öffnung (61) gleitend bewegt werden kann.

6. Wirbelsäule-Osteosynthesevorrichtung nach einem der Ansprüche 1 bis 5,
**dadurch gekennzeichnet, dass**
der Arm (8, 10) mit einer Öffnung (46) versehen ist, die zwischen der Knochenschraube (12) und der Befestigungsschraube (16) angeordnet werden kann.

7. Wirbelsäule-Osteosynthesevorrichtung nach Anspruch 5,
**dadurch gekennzeichnet, dass**
die Befestigungsschraube (16) als Regulierschraube dient.

8. Wirbelsäule-Osteosynthesevorrichtung nach einem der Ansprüche 1 bis 7,
**dadurch gekennzeichnet, dass**
der zweite Arm (10) und die Platte (6) Zentrierflächen (58, 60, 72) aufweisen, die miteinander in Kontakt treten können, um den Arm gegenüber der Platte zu zentrieren.

9. Wirbelsäule-Osteosynthesevorrichtung nach Anspruch 8,
**dadurch gekennzeichnet, dass**
der Arm (10) und die Platte (6) jeweils zwei Zentrierflächen enthalten, die einen Zusammenbau aus Einsteckteilen und Aufnahmeteilen bilden.

10. Wirbelsäule-Osteosynthesevorrichtung nach einem der Ansprüche 8 oder 9,
**dadurch gekennzeichnet, dass**
die Zentrierflächen (58, 60, 72) ebene Flächen sind.

11. Wirbelsäule-Osteosynthesevorrichtung nach einem der Ansprüche 1 bis 10,
**dadurch gekennzeichnet, dass**
die Arme (8, 10) von Hand umgebogen werden können.

## Claims

1. Spinal osteosynthesis system for anterior fixation, comprising a first arm (8, 10) which can receive bone screws (12, 14), and a longitudinal plate (6), the plate (6) being made in one piece with the first arm (8), **characterized in that** it comprises a second arm (8, 10) which can receive bone screws, **in that** the longitudinal plate can rigidly link the arms, and **in that** it comprises means (16, 61) for adjusting a longitudinal position of the second arm (10) on the plate (6) and **in that**, it comprises at least one fixation screw (16) which can be engaged in one of the bone screws (12, 14) associated with one of the arms (8, 10), in order to fix the arm to the bone screw.

2. System according to Claim 1, **characterized in that** the plate (6) has at least one orifice (61) for adjusting the longitudinal position.

3. System according to Claim 2, **characterized in that** the orifice (61) is of oblong shape.

4. System according to Claim 3, **characterized in that** the orifice (61) has, at at least one end, a continuation (69) with a width smaller than the width of a central part of the orifice.

5. System according to Claim 3 or 4, **characterized in that** it includes an adjusting screw (16) which can be fixed to the second arm (10) and can slide in the orifice (61).

6. System according to any one of Claims 1 to 5, **characterized in that** the arm (8, 10) has an orifice (46) which can be interposed between the bone screw (12) and the fixation screw (16).

7. System according to Claim 5, **characterized in that** the fixation screw (16) is the adjusting screw.

8. System according to any one of Claims 1 to 7, **characterized in that** the second arm (10) and the plate (6) have centring faces (58, 60, 72) which can come into mutual contact in order to centre the arm relative to the plate.

9. System according to Claim 8, **characterized in that** the arm (10) and the plate (6) each have two centring faces which can form a male-female assembly.

10. System according to Claim 8 or 9, **characterized in that** the centring faces (58, 60, 72) are plane.

11. System according to any one of Claims 1 to 10, **characterized in that** each arm (8, 10) can be bent manually.
